# EUROPEAN PATENT SPECIFICATION

(11) **EP 2 379 057 B1**
(45) Date of publication and mention of the grant of the patent: **17.07.2013**
(21) Application number: 09795623.9
(22) Date of filing: 11.12.2009
(51) Int. Cl.: A61K 9/20, A61K 31/167, A61K 31/137, A61K 31/135, A61K 31/485, A61K 31/375

(54) **PHENYLEPHRINE FORMULATIONS WITH IMPROVED STABILITY**
PHENYLEPHRIN-FORMULIERUNGEN MIT VERBESSERTER STABILITÄT
COMPOSITIONS DE PHÉNYLÉPHRINE À STABILITÉ AMÉLIORÉE

(30) Priority: 19.12.2008 US 139391 P
(43) Date of publication of application: 26.10.2011
(73) Proprietor: Novartis AG, 4056 Basel (CH)
(72) Inventor: DUNGAN, Timothy, Lincoln, Nebraska 68517 (US); WARRINGTON, Brian, Lincoln, Nebraska 68517 (US)
(74) Representative: Liphardt, Bernd
(86) International application number: PCT/US2009/067618
(87) International publication number: WO 2010/080339

(56) References cited:
- WO-A1-2006/135254
- WO-A1-2007/113536
- WO-A2-2009/023434
- US-A1- 2002 022 057
- US-A1- 2006 127 473

## Description

### Background of the Invention

This application relates to phenylephrine hydrochloride-containing formulations having improved stability and thus increased shelf life.

Phenylephrine hydrochloride (PHL) is a decongestant frequently used in over-the-counter (OTC) cough and cold preparations. PHL is a reactive molecule that undergoes reactions with numerous excipients commonly used in OTC preparations to form other species, with a corresponding decrease in the amount of active PHL in the product. This can lead to a need to set shorter expiration times than may be considered optimum for OTC products.

US2002/0022057 discloses an oral delivery system for pharmaceuticals and *inter alia* describes the encapsulation of PHL via extrusion, using 36.0% saccharose, 36.0% maltodextrin, 0.13% of PHL and 4.2% of water, which can be compressed into tablets that yield 10 mg PHL.

US2006/0127473 discloses stable pharmaceutical compositions comprising unstable active pharmaceutical ingredients and silicified microcrystalline cellulose in a stabilizing effective amount. Phenylephrine is mentioned as an oxygen sensitive API.

While many of the reactions of PHL occur at room temperature and conventional indoor humidity conditions, the reaction rates are higher at elevated temperatures and elevated humidity. OTC preparations intended for sale in Climatic Zones with high ambient temperature and humidity (Climatic Zones 3 (30°C/35% relative air humidity) and 4 (30°C/70% or greater relative air humidity) therefore may have significantly shorter shelf life which may make the product unmarketable in these regions.

### Summary of the Invention

The present invention provides a PHL formulation, preferably in unit dose form, that provides stability sufficient for a shelf life of 24 months, even under high temperature and high humidity conditions. In accordance with the invention, the formulation comprises phenylephrine hydrochloride and maltodextrin in a weight ratio of at least 1:30, and preferably at least 1:40. The formulation also comprises additional active pharmaceutical ingredients (APIs) for cough, cold, and/or congestion, such as diphenhydramine hydrochloride, acetaminophen, dextromethorphan hydrobromide, pheniramine maleate, and chlorpheniramine maleate in conventional amounts relative to the amount of phenylephrine hydrochloride, as well as additional excipients.

A method of making a pharmaceutical granulation is disclosed, performed by combining a pharmaceutical polysaccharide with PHL and processing to form a pharmaceutical granulation. The amount of the polysaccharide must be sufficient to dilute the PHL such that PHL is stable at high temperature and humidity. The weight ratio of phenylephrine hydrochloride to maltodextrin is at least 1:30, and preferably at least 1:40. In addition, the processing may be performed by using a roller compactor-mill-sieve equipment train.

### Detailed Description of the Invention

The present invention provides a way to improve stability of phenylephrine hydrochloride (PHL), for example, in over-the-counter cough and cold preparations. As used in this specification, "stable" means that a composition containing PHL has an assayed value of greater than 95.0% of the labeled content after 24 months in Climatic Zone 3 (30°C/35% relative air humidity).

To practice the invention, maltodextrin is used in sufficient quantities to dilute PHL at a weight ratio at least of 1:30, and preferably at least 1:40.

Conventional manufacturing procedures require the preparation of a composition, which is a powdered or granulated admixture of the active ingredients of a tablet, a binder material and excipients. Without intending to be bound by any particular mechanism, it is believed that the maltodextrin serves as a dry binder and diluent in the formulation, and increases the distance between PHL and reactive excipients or actives.

To arrive at the composition of the present invention, the inventor tested several preparations. The preparations were subjected to high heat and humidity (40°C/75% room humidity) and stability data was collected over six months. This accelerated stability data is an indicator of 24-month real data, based on the Arrhenius equation, which correlates temperature and reaction rate. Preparations utilizing the invention showed PHL stability over six months sufficient to indicate stability for Climate Zones 3 and 4 with a shelf life of 24 months or greater.

In addition, a method of making a pharmaceutical composition is disclosed. To make the pharmaceutical composition, a pharmaceutical polysaccharide and PHL are combined, and then mixed to form a pharmaceutical composition. The amount of polysaccharide must be sufficient to dilute the phenylephrine so that it is stable at high temperature and humidity. The composition should be mixed sufficiently that it is "macroscopically homogeneous," meaning that the active ingredients are substantially evenly dispersed such that a random sample of the composition will contain a proportional amount of each component. The pharmaceutical composition may later be processed into unit dose form.

### Examples

### Example 1

A preparation was made containing 650 mg acetaminophen, 25 mg diphenhydramine hydrochloride, 10 mg phenylephrine hydrochloride, 312 mg maltodextrin, and other excipients per dose. These other excipients included 0.7 mg pharmaceutical-quality dyes, 11.9 mg silicon dioxide, 938 mg natural flavors, 450 mg citric acid, 81 mg sodium citrate, 35 mg calcium phosphate tribasic, and 46 mg high intensity sweeteners. 7500 mg sucrose was added during packaging. The mixture was passed through a roller compactor-mill-sieve equipment train multiple times to achieve particles with an optimal size for further processing. This mixture was further processed into unit dose form.

When this preparation was subjected to high heat and humidity (40°C/75% relative humidity), total phenylephrine hydrochloride degradation (calculated as the % reacted of the labeled content of phenylephrine hydrochloride) was 0.43 at 3 months and 0.81 at 6 months, from an initial degradation of 0.10. In addition, after 24 months storage at 25°C/60% relative humidity, total phenylephrine hydrochloride degradation was calculated to be 0.31. These values correlate to an assay value of not less than 95% of the labeled amount of PHL at the minimum desired shelf life of 24 months in Climate Zones 3 and 4.

### Example 2

A preparation was made containing 650 mg acetaminophen, 20 mg dextromethorphan hydrobromide, 10 mg phenylephrine hydrochloride, 456 mg maltodextrin, and other excipients per dose. These other excipients included 0.7 mg pharmaceutical-quality dyes, 11.9 mg silicon dioxide, 638 mg natural flavors, 705 mg citric acid, 81 mg sodium citrate, 35 mg calcium phosphate tribasic, and 50 mg high intensity sweeteners. 7500 mg sucrose was added during packaging. The mixture was passed through a roller compactor-mill-sieve equipment train multiple times to achieve particles with an optimal size for further processing. This mixture was further processed into unit dose form.

When this preparation was subjected to high heat and humidity (40°C/75% relative humidity), total phenylephrine hydrochloride degradation (calculated as the % reacted of the labeled content of phenylephrine hydrochloride) was 0.44 at 3 months and 0.61 at 6 months, from an initial degradation of 0.21. In addition, after 24 months storage at 25°C/60% relative humidity, total phenylephrine hydrochloride degradation was calculated to be 0.41. These values correlate to an assay value of not less than 95% of the labeled amount of PHL at the minimum desired shelf life of 24 months in Climate Zones 3 and 4.

### Example 3

A preparation is made containing 650 mg acetaminophen, 20 mg pheniramine maleate, 10 mg phenylephrine hydrochloride, 400-600 mg maltodextrin, 50 mg ascorbic acid and other excipients per dose. These other excipients include 0.6-1.58 mg pharmaceutical-quality dyes, 18 mg silicon dioxide, 210-473 mg natural flavors, 650-1000 mg citric acid, 115-1 80 mg sodium citrate, 35 mg calcium phosphate tribasic, 8-50 mg high intensity sweeteners. An additional 14 g sucrose is added during packaging. The pheniramine maleate and phenylephrine hydrochloride are compacted separately. The mixture is passed through a roller compactor-mill-sieve equipment train multiple times to achieve particles with an optimal size for further processing. This mixture is further processed into unit dose form.

## Claims

1. A composition comprising a pharmaceutical polysaccharide and phenylephrine hydrochloride and additional active pharmaceutical ingredients for cough, cold, and/or congestion, selected from diphenhydramine hydrochloride, acetaminophen, dextromethorphan hydrobromide, pheniramine maleate, and chlorpheniramine maleate; wherein the ratio of said polysaccharide to phenylephrine hydrochloride is sufficient to dilute the composition such that phenylephrine hydrochloride is stable at high temperature and humidity; wherein the polysaccharide is maltodextrin and wherein the weight ratio of phenylephrine hydrochloride to maltodextrin is at least 1:30.

2. The composition of claim 1 comprising 650 mg acetaminophen, 25 mg diphenhydramine hydrochloride, 10 mg phenylephrine hydrochloride, and 312 mg maltodextrin per dose.

3. The composition of claim 1 comprising 650 mg acetaminophen, 20 mg dextromethorphan hydrobromide, 10 mg phenylephrine hydrochloride and 456 mg maltodextrin per dose.

4. The composition of claim 1 comprising 650 mg acetaminophen, 20 mg pheniramine maleate, 10 mg phenylephrine hydrochloride, 400-600 mg maltodextrin, and 50 mg ascorbic acid per dose.

## Patentansprüche

1. Zusammensetzung, umfassend ein pharmazeutisches Polysaccharid und Phenylephrin-Hydrochlorid und zusätzliche pharmazeutisch wirksame Bestandteile für Husten, Erkältung und/oder Kongestion, ausgewählt aus Diphenhydramin-Hydrochlorid, Acetaminophen, Dextromethorphan-Hydrobromid, Pheniraminmaleat und Chlorpheniraminmaleat; wobei das Verhältnis des Polysaccharids zu Phenylephrin-Hydrochlorid ausreichend ist, um die Zusammensetzung zu verdünnen, so dass Phenylephrin-Hydrochlorid bei hoher Temperatur und Feuchtigkeit stabil ist; wobei das Polysaccharid Maltodextrin ist und wobei des Gewichtsverhältnis von Phenylephrin-Hydrochlorid zu Maltodextrin mindestens 1:30 ist.

2. Zusammensetzung nach Anspruch 1, umfassend 650 mg Acetaminophen, 25 mg Diphenhydramin-Hydrochlorid, 10 mg Phenylephrin-Hydrochlorid und 312 mg Maltodextrin pro Dosis.

3. Zusammensetzung nach Anspruch 1, umfassend 650 mg Acetaminophen, 20 mg Dextromethorphan-Hydrobromid, 10 mg Phenylephrin-Hydrochlorid und 456 mg Maltodextrin pro Dosis.

4. Zusammensetzung nach Anspruch 1, umfassend 650 mg Acetaminophen, 20 mg Pheniraminmaleat, 10 mg Phenylephrin-Hydrochlorid, 400-600 mg Maltodextrin und 50 mg Ascorbinsäure pro Dosis.

## Revendications

1. Composition comprenant un polysaccharide pharmaceutique et un chlorhydrate de phényléphrine et des ingrédients pharmaceutiques actifs supplémentaires destinés à soigner le rhume, le refroidissement et/ou la congestion, sélectionnés à partir du chlorhydrate de diphénhydramine, de l'acétaminophène, du bromhydrate de dextrométhorphane, du maléate de phéniramine et du maléate de chlorphéniramine, **caractérisée en ce que** le taux dudit polysaccharide par rapport au chlorhydrate de phényléphrine est suffisant pour diluer la composition de sorte que le chlorhydrate de phényléphrine est stable dans des conditions de température et d'humidité élevées ; **caractérisée en ce que** le polysaccharide est de la maltodextrine et **en ce que** le rapport pondéral entre le chlorhydrate de phényléphrine et la maltodextrine est d'au moins 1:30.

2. Composition selon la revendication 1 comprenant 650 mg d'acétaminophène, 25 mg de chlorhydrate de diphénhydramine, 10 mg de chlorhydrate de phényléphrine et 312 mg de maltodextrine par dose.

3. Composition selon la revendication 1 comprenant 650 mg d'acétaminophène, 20 mg de bromhydrate de dextrométhorphane, 10 mg de chlorhydrate de phényléphrine et 456 mg de maltodextrine par dose.

4. Composition selon la revendication 1 comprenant 650 mg d'acétaminophène, 20 mg de maléate de phéniramine, 10 mg de chlorhydrate de phényléphrine, 400-600 mg de maltodextrine et 50 mg d'acide ascorbique par dose.
